**Europäisches Patentamt**

**European Patent Office**

**Office européen des brevets**

(11) Numéro de publication : **0 280 613 B1**

(12) **FASCICULE DE BREVET EUROPEEN**

(45) Date de publication du fascicule du brevet :
**25.03.92 Bulletin 92/13**

(21) Numéro de dépôt : **88400340.1**

(22) Date de dépôt : **16.02.88**

(51) Int. Cl.⁵ : **A61K 31/48, A61K 9/22**

(54) **Comprimé de dihydroergotamine (D.H.E.) du type à matrice hydrophile et son procédé de fabrication.**

(30) Priorité : **18.02.87 FR 8702076**

(43) Date de publication de la demande :
**31.08.88 Bulletin 88/35**

(45) Mention de la délivrance du brevet :
**25.03.92 Bulletin 92/13**

(84) Etats contractants désignés :
**AT BE CH DE ES FR GB GR IT LI LU NL SE**

(56) Documents cités :
**FR-A- 2 327 764**
**FR-A- 2 548 537**
**GB-A- 2 170 407**
**US-A- 4 259 314**
**US-A- 4 608 248**

(73) Titulaire : **PIERRE FABRE MEDICAMENT**
**45, Place Abel Gance**
**F-92100 Boulogne (FR)**

(72) Inventeur : **Sournac, Michel**
**Floralies 4, Impasse Pasteur Beaumont**
**F-63122 Ceyrat (FR)**
Inventeur : **Bougaret, Joel**
**36, boulevard Maréchal Joffre**
**F-81100 Castres (FR)**

(74) Mandataire : **Ahner, Francis et al**
**CABINET REGIMBEAU, 26, avenue Kléber**
**F-75116 Paris (FR)**

**EP 0 280 613 B1**

## Description

La présente invention concerne une nouvelle composition pharmaceutique dont le principe actif comporte un alcaloïde de l'ergot de seigle et dont la formulation galénique permet une libération prolongée dans le temps de ce principe actif.

De telles formes galéniques à libération prolongée permettent notamment d'obtenir un taux constant de principe actif dans l'organisme tout en diminuant la fréquence des prises.

L'état de la technique antérieure peut être illustré par les brevets GB-A-2 170 407 et FR-A-2 327 764.

La demande GB-A-2 170 407 décrit des compositions sous forme de comprimés comprenant un principe actif à base de dihydroergotamine dans une matrice contenant, à titre de composant essentiel, un matériau gras inerte associé à une substance gonflante hydrophile. La mise en oeuvre de ces compositions nécessite une granulation avec fusion de l'excipient gras pour obtenir des grains essentiellement destinés à une mise en gélule.

La demande FR-A-2 327 764 décrit des compositions, par exemple des comprimés, comprenant une dispersion d'alcaloïdes d'ergot de seigle dans une matrice comprenant un polymère solide et des agents diluants.

Des formes à libération prolongée pour l'administration de médicaments à base notamment d'un alcaloïde de l'ergot de seigle ont déjà été réalisées ; parmi celles-ci, la forme gélules à base de microgranules chronodialysants (brevet FR 2 477 015) s'est révélée être particulièrement performante ; ces gélules permettent en effet la libération prolongée du principe actif, à savoir le méthane-sulfonate de dihydroergotamine, selon un profil cinétique assurant la libération d'un taux constant du principe actif pendant une période d'au moins 8 heures.

Cette cinétique de libération est obtenue grâce à un procédé très complexe de fabrication de microgranules. Il est nécessaire, en particulier, de recourir à plusieurs enrobages des microgranules inclus dans ces gélules. Parmi ces enrobages, certains doivent être gastro et/ou entero-solubles et d'autres gastro et/ou entero-resistants et ce, dans une répartition judicieuse de façon à établir un équilibre permettant un délitement programmé en fonction du pH. Ceci implique la mise en oeuvre d'un matériel coûteux et complexe, le recours à une main d'oeuvre hautement spécialisée et un cycle de fabrication très long. Au surplus, les gélules obtenues ont des dimensions relativement importantes, ce qui peut rendre leur absorption désagréable.

On a trouvé de façon surprenante qu'une formulation de méthane-sulfonate de dihydroergotamine, présentée sous la forme d'un comprimé du type à matrice hydrophile, possédait des caractéristiques de libération prolongée équivalentes à celles obtenues avec la forme microgranules en gélule, sans en avoir les inconvénients de fabrication et d'utilisation. Cette identité de libération est obtenue bien qu'il n'y ait pas, dans la nouvelle formulation du comprimé selon la présente invention, une association d'une dose unitaire instantanée avec une dose unitaire à libération prolongée.

La présente invention se rapporte à un comprimé du type comportant une dispersion homogène d'un principe actif à base de dihydroergotamine (D.H.E.) ou de l'un de ses dérivés dans une matrice hydrophile, caractérisé en ce que ladite matrice est pratiquement exempte de composé gras inerte, à l'exception de faibles quantités inférieures ou égales à 1% de stéarate de magnésium et/ou d'acide stéarique utilisés dans le seul but de lubrifier le mélange, et comporte au moins un composant gonflant comprenant une ou plusieurs substances hydrophiles choisies parmi les dérivés alginiques, les gommes naturelles, la méthylcellulose, la carboxyméthylcellulose, l'hydroxypropylcellulose et/ou la méthylhydroxypropylcellulose, et un composant diluant dans le rapport gonflant/diluant compris entre 0,2 et 0,6 de préférence égal à 0,4, ledit composant diluant comprenant au moins un dérivé d'amidon, et en ce que le principe actif est présent à raison de 2 à 20°% en poids par rapport au poids total dudit comprimé.

Les alcaloïdes de l'ergot de seigle utilisés dans le cadre de la présente invention sont la dihydroergotamine (D.H.E.) et ses dérivés; on utilisera de préférence le mono-méthane-sulfonate de dihydroergotamine.

Il est particulièrement avantageux d'avoir recours à des comprimés comportant 6% en poids de principe actif par rapport au poids total du comprimé.

Le composant gonflant comporte de préférence une ou plusieurs substances polymères hydrophiles de viscosité apparente, à 2% poids/poids à 20°C, comprise entre 0,1 et 30 Pa.s. La viscosité est mesurée à l'aide de tubes d'UBBELHODE.

Le polymère hydrophile tout particulièrement préféré est la méthylhydroxypropylcellulose de viscosité égale à environ 15 Pa.s.

Parmi les gommes naturelles, on utilise de préférence la carraghénine.

Ces polymères peuvent être utilisés seuls ou en association entre eux.

Le composant diluent est choisi parmi des substances hydrophiles solubles telles que le lactose, le sorbitol et/ou le mannitol, ou parmi des substances d'hydrosolubilité moindre ou nulle telles que les dérivés des amidons, les phosphates ou sulfates de calcium et/ou la silice colloïdale.

On utilise de préférence en association deux ou plusieurs diluants choisis dans chacune de ces catégories, de façon à garantir une bonne cohésion de la matrice, lors de son contact avec le milieu liquide.

Les comprimés selon la présente invention comportent en outre un agent lubrifiant, tel que du stéarate de magnésium.

Les comprimés selon la présente invention se caractérisent aussi par la simplicité de leur réalisation industrielle.

Leur procédé de fabrication est, en effet, caractérisé par les opérations successives suivantes :
1) tamisage des différents composants et du principe actif,
2) mélange des différents composants et du principe actif pendant environ 20 minutes,
3) ajout d'un lubrifiant et mélange pendant environ 10 minutes,
4) compression sur une machine rotative de production.

Un tel procédé de fabrication, caractérisé par un mélange à sec des divers composants, suivi d'une compression direct, confère une grande stabilité mécanique aux comprimés, tout en évitant un grand nombre d'opérations additionnelles telles que la granulation ou le mouillage, et élimine de ce fait tout risque de dégradation du principe actif.

Ainsi, l'ensemble de ces éléments permet une très bonne stabilité physicochimique du principe actif, tout en assurant une parfaite cohésion de la matrice hydrophile et en garantissant le respect des normes de fabrication puis de contrôle, pour chacun des lots industriels reproduits, en accord avec le prototype que l'on s'est imposé.

L'exacte équivalence entre la forme gélule et la forme comprimé a été démontrée par des essais biopharmaceutiques. En effet, l'étude comparative entre les deux formes, de quantités libérées de principe actif en fonction du temps, permet d'établir une équivalence de libération, tant pour la fraction instantanée que pour la fraction à libération prolongée. Le contrôle des phases de libération est permis par une sélection appropriée tant des composants hydrophiles, que du rapport pondéral entre le mono-méthane-sulfonate de dihydroergotamine et cette même substance hydrophile.

Une équivalence in vitro, entre les deux formes a été démontrée sur un dispositif à bouteilles tournantes, par exemple du type "Diffutest" de la Société Eurand. Un tel appareil est essentiellement une enceinte thermostatée réglée à 37° ± 0,5°C et contenant une roue munie de 8 logements à ressorts destinés à soutenir des flacons pendant la rotation ; la roue est prévue pour tourner à la vitesse fixe de 30 tours/minute. Il est muni de flacons en verre de 45 ml, formés de deux parties :
– 1 tube en verre ouvert aux deux extrémités, sur lequel on ajuste à la partie inférieure,
– un fritté, lui-même recouvert d'un filtre en intissé.

La partie supérieure du tube en verre et le fritté sont bouchés à l'émeri.

Le produit à tester est introduit dans ces flacons, ainsi que la quantité de solution nécessaire (45 ml) pour chaque phase de l'analyse. Ensuite, chaque flacon hermétiquement clos est placé sur le porte tube rotatif, on fait tourner celui-ci à vitesse constante durant la période nécessaire pour chaque phase de l'analyse.

A la fin de chaque période de rotation, le liquide contenu dans le flacon est transféré dans un récipient adéquat par filtration au moyen d'une trompe à vide.

Cette démonstration a été effectuée dans des conditions de variation de pH, et avec une adaptation permettant le maintien de la forme galénique dans le liquide d'épreuve pour éviter tout collage sur le fritté.

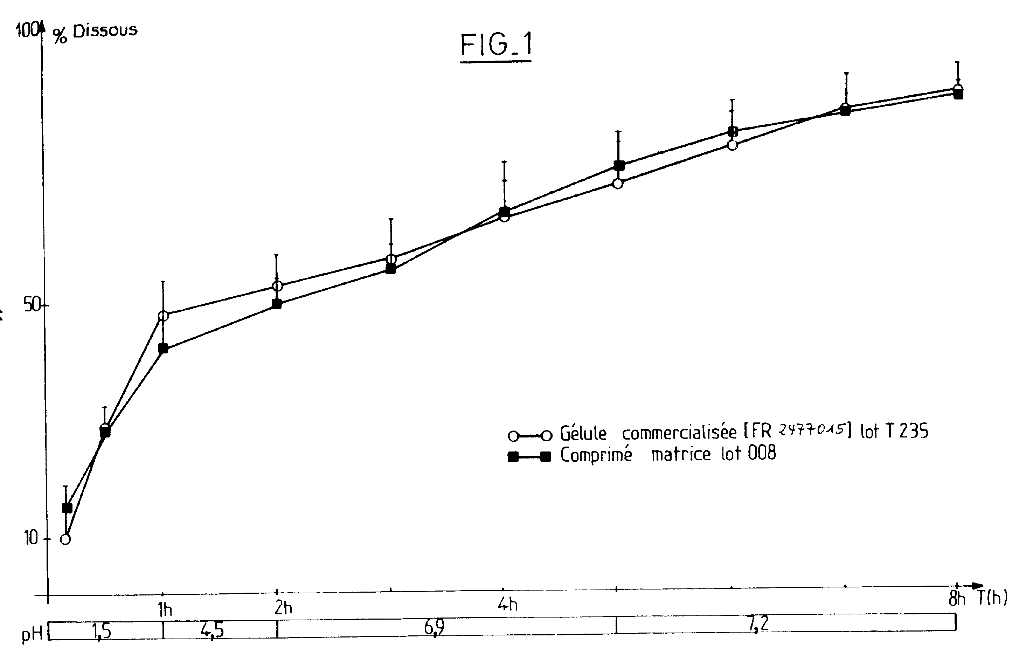

La figure 1 représente les pourcentages de principe actif dissous, en fonction du temps et du pH, des deux formes galéniques comparées :
○—○ représente la forme gelule commercialisée (Brevet FR-A-2 477 015)
■—■ représente la forme comprimé à matrice hydrophile selon la présente invention.

Dans les conditions de réalisation de ces tests, l'identité des profils de libération du principe actif obtenus pour les formes gélule et comprimé est vérifiée, et les pourcentages de principe actif libéré en fonction du temps sont les suivants :

$$5\% \leqslant 0,17 \text{ h} \leqslant 20\%$$
$$30\% \leqslant 1 \text{ h} \leqslant 60\%$$
$$50\% \leqslant 4 \text{ h} < 70\%$$
$$8 \text{ h} \geqslant 70\%$$

le profil recherché (forme de référence : gélule commercialisée (brevet FR-A-2 477 015) est ainsi obtenu par la forme comprimé à libération prolongée selon la présente invention.

Différents comprimés peuvent ainsi être réalisés, comme illustré par les exemples suivants :

EXEMPLE 1

```
dihydroergotamine mono-méthane-sulfonate ...............    5 mg
lactose .................................................   34 mg
amidon de maïs prégélatinisé ............................   18 mg
méthylhydroxypropylcellulose 15 Pa.s ....................   20 mg
silice colloïdale .......................................    0,5 mg
stéarate de magnésium ...................................    0,5 mg
pour un comprimé terminé à ..............................   78 mg
```

EXEMPLE 2

```
dihydroergotamine mono-méthane-sulfonate ...............   10 mg
méthylcellulose 4 Pa.s .................................   40 mg
amidon de maïs prégélatinisé ...........................   46 mg
lactose ................................................   59 mg
stéarate de magnésium ..................................    1 mg
pour un comprimé terminé à .............................  156 mg
```

EXEMPLE 3

```
dihydroergotamine mono-méthane-sulfonate ...............   20 mg
lactose ................................................  130 mg
amidon de maïs..........................................   72 mg
méthylhydroxypropylcellulose 15 Pa.s ...................   86 mg
silice colloïdale ......................................    2 mg
stéarate de magnésium ..................................    2 mg
pour un comprimé terminé à .............................  312 mg
```

**Revendications**

**Revendications pour les Etats contractants suivants: DE, GB, FR, IT, NL, SE, CH, BE, UT, LU, LI**

1. Comprimé du type comportant une dispersion homogène d'un principe actif à base de dihydroergotamine (D.H.E.) ou de l'un de ses dérivés dans une matrice hydrophile, caractérisé en ce que ladite matrice est pratiquement exempte de composé gras inerte, à l'exception de faibles quantités inférieures ou égales à 1% de stéarate de magnésium et/ou d'acide stéarique utilisés dans le seul but de lubrifier le mélange, et comporte au moins un composant gonflant comprenant une ou plusieurs substances hydrophiles choisies parmi les dérivés alginiques, les gommes naturelles, la méthylcellulose, la carboxyméthylcellulose, l'hydroxypropylcellulose et/ou la méthylhydroxypropylcellulose, et un composant diluant dans le rapport gonflant/diluant compris entre 0,2 et 0,6 de préférence égal à 0,4, ledit composant diluant comprenant au moins un dérivé d'amidon, et en ce que le principe actif est présent à raison de 2 à 20% en poids par rapport au poids total dudit comprimé.

2. Comprimé selon la revendication 1, caractérisé en ce que le principe actif est présent à raison de 6% en poids par rapport au poids total dudit comprimé.

3. Comprimé selon l'une quelconque des revendications 1 et 2, carctérisé en ce que le composant gonflant comporte une ou plusieurs substances polymères hydrophiles de viscosité apparente comprise entre 0,1 et 30 Pa.s.

4. Comprimé selon l'une des revendications 1 à 3, caractérisé en ce que la substance polymère est de la méthylhydroxypropylcellulose dont la viscosité est de 15 Pa.s.

5. Comprimé selon l'une quelconque des revendications 1 à 4, caractérisé en ce que le composant diluant comporte en outre une ou plusieurs substances choisies parmi le lactose, le sorbitol, le mannitol, les phosphates ou sulfates de calcium, la silice colloïdale.

6. Comprimé selon l'une quelconque des revendications 1 à 5, caractérisé en ce qu'il comporte en outre un lubrifiant.

7. Comprimé selon la revendication 6, caractérisé en ce que le lubrifiant est l'acide stéarique ou l'un de ses dérivés tels que le stéarate de magnésium.

8. Procédé selon l'une quelconque des revendications 1 à 7, caractérisé en ce que J'on prépare la composition suivante :

| | |
|---|---|
| - mono-méthane-sulfonate de D.H.E. | 5 mg |
| - Méthylhydroxypropylcellulose, 15 Pa.s. | 20 mg |
| - Lactose | 34 mg |
| - Amidon de maïs prégélatinisé | 18 mg |
| - Silice colloïdale | 0,50 mg |
| - Stéarate de magnésium | 0,50 mg |
| pour un comprimé terminé de | 78 mg. |

9. Procédé de fabrication d'un comprimé selon l'une quelconque des revendications 1 à 8, caractérisé en ce qu'il comporte les opérations successives suivantes :
1) tamisage des différents composants, gonflant et diluant, et du principe actif;
2) mélange de ces composants, gonflant et diluant, et du principe actif à raison d'un rapport pondéral gonflant/diluant compris entre 0,2 et 0,6 et d'une quantité pondérale de principe actif comprise entre 2 et 20% par rapport au poids total du comprimé;
3) ajout d'un lubrifiant et mélange pendant 10 minutes;
4) compression sur une machine rotative de production.

**Revendications pour les Etats contractants suivants: ES, GR**

1. Procédé de fabrication d'un comprimé du type comportant une dispersion homogène d'un principe actif à base de dihydroergotamine (D.H.E.) ou de l'un de ses dérivés dans une matrice hydrophile pratiquement exempte de composé gras inerte, à l'exception de faibles quantités inférieures ou égales à 1% de stéarate de magnésium et/ou d'acide stéarique utilisés dans le seul but de lubrifier le mélange, et comportant au moins un composant gonflant comprenant une ou plusieurs substances hydrophiles choisies parmi les dérivés alginiques, les gommes naturelles, la méthylcellulose, la carboxyméthylcellulose, l'hydroxypropylcellulose et/ou la méthylhydroxypropylcellulose, et un composant diluant dans le rapport gonflant/diluant compris entre 0,2 et 0,6 de préférence égal à 0,4, ledit composant diluant comprenant au moins un dérivé d'amidon, le principale actif étant présent à raison de 2 à 20% en poids par rapport au poids total dudit comprimé, caractérisé en ce qu'il comporte les opérations successives suivantes :
1) tamisage des différents composants, gonflant et diluant, et du principe actif;
2) mélange de ces composants, gonflant et diluant, et du principe actif à raison d'un rapport pondéral gonflant/diluant compris entre 0,2 et 0,6 et d'un quantité pondérale de principe actif comprise entre 2 et 20% par rapport au poids total du comprimé;

3) ajout d'un lubrifant et mélange pendant 10 minutes;

4) compression sur une machine rotative de production.

2. Procédé selon la revendication 1, caractérisé en ce que le principe actif est présent à raison de 6% en poids par rapport au poids total dudit comprimé.

3. Procédé selon l'une quelconque des revendications 1 et 2, caractérisé en ce que le composant gonflant comporte une ou plusieurs substances polymères hydrophiles de viscosité apparente comprise entre 0,1 et 30 Pa.s.

4. Procédé selon l'une des revendications 1 à 3, caractérisé en ce que la substance polymère est de la méthylhydroxypropylcellulose dont la viscosité est de 15 Pa.s.

5. Procédé selon l'une des revendications 1 à 4, caractérisé en ce que le composant diluant comporte en outre une ou plusieurs substances choisies parmi le lactose, le sorbitol, le mannitol, les phosphates ou sulfates de calcium, la silice colloïdale.

6. Procédé selon l'une quelconque des revendications 1 à 5, caractérisé en ce que l'on ajoute en outre un lubrifiant.

7. Procédé selon la revendication 6, caractérisé en ce que le lubrifiant est l'acide stéarique ou l'un de ses dérivés tels que le stéarate de magnésium.

8. Procédé selon l'une quelconque des revendications 1 à 7, caractérisé en ce que l'on prépare la composition suivante :

| | |
|---|---|
| - mono-méthane-sulfonate de D.H.E. | 5 mg |
| - Méthylhydroxypropylcellulose, 15 Pa.s. | 20 mg |
| - Lactose | 34 mg |
| - Amidon de maïs prégélatinisé | 18 mg |
| - Silice colloïdale | 0,50 mg |
| - Stéarate de magnésium | 0,50 mg |
| pour un comprimé terminé de | 78 mg. |

## Patentansprüche

**Patentansprüche für folgende Vertragsstaaten: AT, BE, CH, DE, FR, GB, IT, LI, LU, NL, SE**

1. Tablette des Typs, der eine homogene Dispersion eines aktiven Prinzips (Wirkstoffes) auf Basis von Dihydroergotamin (D.H.E.) oder eines seiner Derivate in einer hydrophilen Matrix umfaßt, dadurch gekennzeichnet, daß diese Matrix praktisch frei von einer inerten Fett-Verbindung ist mit Ausnahme von geringen Mengen von höchstens 1 % Magnesiumstearat und/oder Stearinsäure, die nur zu dem Zweck verwendet werden, die Mischung gleitfähig zu machen, und umfaßt mindestens einen Quellungsmittel-Bestandteil, der enthält eine oder mehr hydrophile Substanzen, ausgewählt aus den Alginderivaten, Naturharzen (Naturgummis), Methylcellulose, Carboxymethylcellulose, Hydroxypropylcellulose und/oder Methylhydroxypropylcellulose, und einen Verdünnungsmittel-Bestandteil in einem Verhältnis Quellungsmittel/Verdünnungsmittel zwischen 0,2 und 0,6, vorzugsweise von 0,4, wobei der Verdünnungsmittel-Bestandteil mindestens ein Stärkederivat enthält, und daß das aktive Prinzip (der Wirkstoff) in einer Menge von 2 bis 20 Gew.-%, bezogen auf das Gesamtgewicht der Tablette, vorliegt.

2. Tablette nach Anspruch 1, dadurch gekennzeichnet, daß das aktive Prinzip (der Wirkstoff) in einer Menge von 6 Gew.-%, bezogen auf das Gesamtgewicht der Tablette, vorliegt.

3. Tablette nach einem der Ansprüche 1 und 2, dadurch gekennzeichnet, daß der Quellungsmittel-Bestandteil eine oder mehr hydrophile polymere Substanzen mit einer scheinbaren Viskosität zwischen 0,1 und 30 Pa.s umfaßt.

4. Tablette nach einem der Ansprüche 1 bis 3, dadurch gekennzeichnet, daß es sich bei der polymeren Substanz um die Methylhydroxypropylcellulose handelt, deren Viskosität 15 Pa.s beträgt.

5. Tablette nach einem der Ansprüche 1 bis 4, dadurch gekennzeichnet, daß die Verdünnungsmittel-Komponente außerdem ein oder mehr Substanzen, ausgewählt aus Lactose, Sorbit, Mannit, den Phosphaten oder Sulfaten von Calcium und kolloidalem Siliciumoxid umfaßt.

6. Tablette nach einem der Ansprüche 1 bis 5, dadurch gekennzeichnet, daß sie außerdem ein Gleitmittel enthält.

7. Tablette nach Anspruch 6, dadurch gekennzeichnet, daß es sich bei dem Gleitmittel um die Stearinsäure oder eines ihrer Derivate, wie z.B. Magnesiumstearat, handelt.

8. Tablette nach einem der Ansprüche 1 bis 7, welche die folgende Zusammensetzung aufweist:

| | | |
|---|---|---|
| – Monomethansulfonat von D.H.E. | 5 | mg |
| – Methylhydroxypropylcellulose, 15 Pa.s | 20 | mg |
| – Lactose | 34 | mg |
| – vorgelatinierte Maisstärke | 18 | mg |
| – kolloidales Siliciumdioxid | 0,50 | mg |
| – Magnesiumstearat | 0,50 | mg |
| für eine fertige Tablette von | 78 | mg |

9. Verfahren zur Herstellung einer Tablette nach einem der Ansprüche 1 bis 8, dadurch gekennzeichnet, daß es die nachstehenden aufeinanderfolgenden Arbeitsgänge umfaßt:

(1) das Sieben der verschiedenen Bestandteile, des Quellungsmittels und des Verdünnungsmittels und des aktiven Prinzips (Wirkstoffs);

(2) das Mischen dieser Bestandteile, des Quellungsmittels und des Verdünnungsmittels und des aktiven Prinzips (Wirkstoffes) in einem Gewichtsmengenverhältnis Quellungsmittel/Verdünnungsmittel zwischen 0,2 und 0,6 und in einer Gewichtsmenge des aktiven Prinzips (Wirkstoffes) zwischen 2 und 20 %, bezogen auf das Gesamtgewicht der Tablette;

(3) die Zugabe eines Gleitmittels und das 10-minütige Mischen; und

(4) das Pressen auf einer sich drehenden Produktionsvorrichtung.

**Patentansprüche für folgende Vertragsstaaten: ES, GR**

1. Verfahren zur Herstellung einer Tablette des Typs, der umfaßt eine homogene Dispersion eines aktiven Prinzips (Wirkstoffes) auf Basis von Dihydroergotamin (D.H.E.) oder eines seiner Derivate in einer hydrophilen Matrix, die praktisch frei von einer inerten Fettverbindung ist mit Ausnahme von geringen Mengen von höchstens 1 % Magnesiumstearat und/oder Stearinsäure, die nur für den Zweck verwendet werden, die Mischung gleitfähig zu machen, und die umfaßt mindestens einen Quellungsmittel-Bestandteil, der ein oder mehr hydrophile Substanzen, ausgewählt aus den Alginderivaten, den Naturharzen (Naturgummis), Methylcellulose, Carboxymethylcellulose, Hydroxypropylcellulose und/oder Methylhydroxypropylcellulose, enthält, und einen Verdünnungsmittl-Bestandteil im Verhältnis Quellungsmittel/Verdünungsmittel zwischen 0,2 und 0,6, vorzugsweise von 0,4, wobei der Verdünnungsmittel-Bestandteil mindestens ein Stärkederivat enthält, wobei das aktive Prinzip (der Wirkstoff) in einer Menge von 2 bis 20 Gew.-%, bezogen auf das Gesamtgewicht der Tablette, vorliegt, dadurch gekennzeichnet, das es die nachstehend angegebenen aufeinanderfolgenden Arbeitsgänge umfaßt:

(1) das Sieben der verschiedenen Bestandteile, des Quellungsmittels und des Verdünnungsmittels und des aktiven Prinzips (des Wirkstoffes);

(2) das Mischen dieser Bestandteile, des Quellungsmittels und des Verdünnungsmittels und des aktiven Prinzips (des Wirkstoffes) in einem Gewichtsverhältnis Quellungsmittel/Verdünnungsmittel zwischen 0,2 und 0,6 und des aktiven Prinzips (Wirkstoffes) in einer Gewichtsmenge zwischen 2 und 20 %, bezogen auf das Gesamtgewicht der Tablette;

(3) die Zugabe eines Gleitmittels und das 10-minütige Mischen; und

(4) das Pressen auf einer sich drehenden Produktions-Vorrichtung.

2. Verfahren nach Anspruch 1, dadurch gekennzeichnet, daß das aktive Prinzip (der Wirkstoff) in einer Menge von 6 Gew.%, bezogen auf das Gesamtgewicht der Tablette, vorliegt.

3. Verfahren nach einem der Ansprüche 1 und 2, dadurch gekennzeichnet, daß der Quellungsmittel-Bestandteil eine oder mehr hydrophile polymere Substanzen mit einer scheinbaren Viskosität zwischen 0,1 und 30 Pa.s umfaßt.

4. Verfahren nach einem der Ansprüche 1 bis 3, dadurch gekennzeichnet, daß es sich bei der polymeren Substanz um die Methylhydroxypropylcellulose handelt, deren Viskosität 15 Pa.s beträgt.

5. Verfahren nach einem der Ansprüche 1 bis 4, dadurch gekennzeichnet, daß der Verdünnungsmittel-Bestandteil eine oder mehr Substanzen, ausgewählt aus Lactose, Sorbit, Mannit, den Phosphaten oder Sulfaten von Calcium und kolloidalem Siliciumdioxid, umfaßt.

6. Verfahren nach einem der Ansprüche 1 bis 5, dadurch gekennzeichnet, daß man außerdem ein Gleitmittel zugibt.

7. Verfahren nach Anspruch 6, dadurch gekennzeichnet, daß es sich bei dem Gleitmittel um Stearinsäure oder eines ihrer Derivate, wie z.B. Magnesiumstearat, handelt.

8. Verfahren nach einem der Ansprüche 1 bis 7, dadurch gekennzeichnet, daß man die folgende Zusammensetzung herstellt:

| | | |
|---|---:|---|
| – Monomethansulfonat von D.H.E. | 5 | mg |
| – Methylhydroxypropylcellulose, 15 Pa.s | 20 | mg |
| – Lactose | 34 | mg |
| – Methylhydroxypropylcellulose, 15 Pa.s | 20 | mg |
| – Lactose | 34 | mg |
| – vorgelatinierte Maisstärke | 18 | mg |
| – kolloidales Siliciumdioxid | 0,50 | mg |
| – Magnesiumstearat | 0,50 | mg |
| für eine fertige Tablette von | 78 | mg |

## Claims

**Claims for the following Contracting States: AT, BE, CH, DE, FR, GB, IT, LI, LU, NL, SE**

1. Tablet of the type comprising a homogeneous dispersion of an active principle based on dihydroergotamine (D.H.E.) or one of its derivatives in a water-soluble matrix, characterized in that said matrix is substantially devoided of inert fatty compound, with the exception of small amount, which are lower or equal to 1%, of magnesium stearate and/or stearic acid, used only in the purpose of lubricating the mixture, and comprises at least a swelling agent comprising one or more water-soluble polymeric substances chosen from among alginic acid derivatives, naturally occurring gums, methylcellulose, carboxymethylcellulose, hydroxypropylcellulose, and/or methylhydroxypropylcellulose, and a diluent in a ratio of swelling agent/diluent of between 0.2 and 0.6 and preferably equal to 0.4, the said diluent comprising at least one starch derivative, and in that the active principle is present at a concentration of 2 to 20 % by weight of the total weight of the said tablet.

2. Tablet according to Claim 1, characterized in that the active principle is present at a concentration of 6 % by weight of the total weight of the said tablet.

3. Tablet according to any one of the Claims 1 and 2, characterized in that the swelling agent comprises one or more water-soluble polymeric substances of apparent viscosity varying between 0.1 and 30 Pa.s.

4. Tablet according to any one of Claims 1 to 3, characterized in that the polymeric substance is methylhydroxypropylcellulose, the viscosity of which is 15 Pa.s.

5. Tablet according to any one of the Claims 1 to 4, characterized in that the diluent comprises, in addition, one or several substances chosen from among lactose, sorbitol, mannitol, the phosphates or sulfates of calcium, colloidal silica.

6. Tablet according to any one of the Claims 1 to 5, characterized in that it contains in addition a lubricant.

7. Tablet according to Claim 6, characterized in that the lubricant is stearic acid or one of its derivatives such as magnesium stearate.

8. Tablet according to any one of the Claims 1 to 7 possessing the following composition:

| | |
|---|---|
| - dihydroergotamine mono-methane-sulfonate | 5 mg |
| - methylhydroxypropylcellulose, 15 Pa.s | 20 mg |
| - lactose | 34 mg |
| - pre-gelatinized maize starch | 18 mg |
| - colloidal silica | 0.50 mg |
| - magnesium stearate | 0.50 mg |
| - for a finished tablet of | 78 mg. |

9. Manufacturing process for a tablet according to any one of the Claims 1 to 8, characterized in that it comprises the following sequence of operations :

1) sieving of the different components, swelling agent, diluent and active principle;

2) mixing of these components, swelling agent, diluent and the active principle in a weight ratio of swelling agent/diluent of between 0.2 and 0.6 and a concentration of active principle varying between 2 and 20 % by weight of the total weight of the tablet;

3) addition of a lubricant and mixing for 10 minutes;

4) compression in a rotary pelleting manufacturing machine.

**Claims for the following Contracting States: ES, GR**

1. Manufacturing process for a tablet of the type comprising a homogeneous dispersion of an active principle based on dihydroergotamine (D.H.E.) or one of its derivatives in a water-soluble matrix, substantially devoided of inert fatty compound, with the exception of small amount, which are lower or equal to 1%, of magnesium stearate and/or stearic acid, used only in the purpose of lubricating the mixture, and comprising at least a swelling agent which comprises one or more water-soluble polymeric substances chosen from among alginic acid derivatives, naturally occurring gums, methylcellulose, carboxymethylcellulose, hydroxypropylcellulose, and/or methylhydroxypropylcellulose, and a diluent in a ratio of swelling agent/diluent of between 0.2 and 0.6 and preferably equal to 0.4, the said diluent comprising at least one starch derivative, the active principle being present at a concentration of 2 to 20 % by weight of the total weight of the said tablet, characterized in that it comprises the following sequence of operations:

1) sieving of the different components, swelling agent, diluent and active principle;

2) mixing of these components, swelling agent, diluent and the active principle in a weight ratio of swelling agent/diluent of between 0.2 and 0.6 and a concentration of active principle varying between 2 and 20 % by weight of the total weight of the tablet;

3) addition of a lubricant and mixing for about 10 minutes;

4) compression in a rotary pelleting manufacturing machine.

2. Process according to Claim 1, characterized in that the active principle is present at a concentration of 6 % by weight of the total weight of the said tablet.

3. Process according to any one of the Claims 1 and 2, characterized in that the swelling agent comprises one or more water-soluble polymeric substances of apparent viscosity varying between 0.1 and 30 Pa.s.

4. Process according to any one of Claims 1 to 3, characterized in that the polymeric substance is methylhydroxypropylcellulose, the viscosity of which is 15 Pa.s.

5. Process according to any one of the Claims 1 to 4, characterized in that the diluent comprises, in addition, one or several substances chosen from among lactose, sorbitol, mannitol, the phosphates or sulfates of calcium, colloidal silica.

6. Process according to any one of the Claims 1 to 5, characterized in that it contains in addition a lubricant.

7. Process according to Claim 6, characterized in that the lubricant is stearic acid or one of its derivatives such as magnesium stearate.

8. Process according to any one of the Claims 1 to 7 possessing the following composition:

| | | |
|---|---|---|
| - dihydroergotamine mono-methane-sulfonate | 5 | mg |
| - methylhydroxypropylcellulose, 15 Pa.s | 20 | mg |
| - lactose | 34 | mg |
| - pre-gelatinized maize starch | 18 | mg |
| - colloidal silica | 0.50 | mg |
| - magnesium stearate | 0.50 | mg |
| - for a finished tablet of | 78 | mg. |

FIG.1

Gélule commercialisée [FR 2477045] lot T 235
Comprimé matrice lot 008